(19) Europäisches Patentamt
European Patent Office
Office européen des brevets

(11) **EP 4 075 168 A1**

(12) **EUROPEAN PATENT APPLICATION**

(43) Date of publication:
**19.10.2022 Bulletin 2022/42**

(21) Application number: **22168511.8**

(22) Date of filing: **14.04.2022**

(51) International Patent Classification (IPC):
**G01T 1/17** (2006.01)     **G01T 1/29** (2006.01)

(52) Cooperative Patent Classification (CPC):
**G01T 1/2985; G01T 1/17**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.04.2021 US 202117230372**
**02.02.2022 JP 2022014935**

(71) Applicant: **Canon Medical Systems Corporation Tochigi 324-0036 (JP)**

(72) Inventors:
• **QI, Wenyuan**
  **Tochigi (JP)**
• **QIANG, Yi**
  **Tochigi (JP)**
• **PENG, Peng**
  **Tochigi (JP)**
• **ASMA, Evren**
  **Tochigi (JP)**
• **KOLTHAMMER, Jeffrey**
  **Tochigi (JP)**

(74) Representative: **Marks & Clerk LLP**
**15 Fetter Lane**
**London EC4A 1BW (GB)**

(54) **NUCLEAR MEDICINE DIAGNOSIS DEVICE AND NUCLEAR MEDICINE IMAGE DATA GENERATION METHOD**

(57)     A nuclear medicine diagnosis device according to an embodiment includes an acquisition unit 770a, a determination unit 770b, and a generation unit 770c. The acquisition unit 770a acquires gamma ray detection data of an object. The determination unit 770b determines the weight of each of multiple coincidences based on three or more of events detected within a first time window, in the gamma ray detection data. The generation unit 770c generates first nuclear medicine image data based on the weight.

FIG.3

EP 4 075 168 A1

## Description

BACKGROUND

The background description provided herein is for the purpose of generally presenting the context of the disclosure. Work of the presently named inventors, to the extent the work is described in this background section, as well as aspects of the description that may not otherwise qualify as prior art at the time of filing, are neither expressly nor impliedly admitted as prior art against the present disclosure.

Positron emission tomography (PET) device is a functional imaging modality that is capable of imaging biochemical processes in humans or animals through the use of radioactive tracers. In PET imaging, a tracer agent is administered to the patient (object) to be imaged via injection, inhalation, or ingestion. After administration of the tracer agent, the physical and bio-molecular properties of the agent cause the agent to concentrate at specific locations in the patient's body. The actual spatial distribution of the agent, the intensity of the region of accumulation of the agent, and the kinetics of the process from administration to its eventual elimination are all factors that may have clinical significance.

During this process, a tracer attached to the agent will emit positrons. When an emitted positron collides with an electron, an annihilation event occurs, wherein the positron and electron are combined. Most of the time, an annihilation event produces two gamma rays (at 511 keV) traveling at substantially 180 degrees apart. The two gamma rays, each known as a single, are detected by detector elements to produce a pair of coincidences. However, measured coincidences include both true coincidences and random coincidences.

In PET scanners, the singles-pairing can be performed with hardware coincidence circuitry, where a multi-photon coincidence event (i.e., more than two singles that are in coincidence) are often rejected and only two-photon coincidence events meeting strict criteria are accepted. With a higher count rate, the multi-photon coincidence event rate will increase significantly, and simply rejecting all the multi-photon coincidence events can lead to massive loss of true coincidence events. Therefore, it is better to retain the multi-photon coincidence events so as to increase the noise equivalent count rate (NECR). While both methods of wholly accepting or rejecting all multi-photon coincidence events yields similar image quality, random events and scatter events are also increased by accepting all multi-photon coincidence events, which leads to collateral degradation of image data. Thus, a method to identify and select the true coincidences from among the set of multi-coincidences is desired. Consequently, it is desired that identifying and selecting the true coincidences from among the set of multi-coincidences leads to generation of nuclear medicine image data with good image quality.

Summary of Invention

A nuclear medicine diagnosis device provided in one aspect of the present invention includes an acquisition unit, a determination unit, and a generation unit. The acquisition unit acquires gamma ray detection data of an object. The determination unit determines the weight of each of multiple coincidences based on three or more of events detected within a first time window, in the gamma ray detection data. The generation unit generates first nuclear medicine image data based on the weight.

The determination unit may also determine the weight based on the line of response "LOR" defined by the two events within a second time window including the two events.

The determination unit may also determine the weight based on the frequency of the LOR defined by the two events.

The determination unit may also determine the weight such that the weight increases as the frequency increases.

The determination unit may also determine the weight based on second nuclear medicine image data generated based on the LOR defined by the two events.

The determination unit may also determine the weight of each of the coincidences based on the intensities of voxels forming the second nuclear medicine image data and through which each of a plurality of the LORs defined by three or more of the events passes.

The determination unit may also determine the weight of each of the coincidences based on the intensities of voxels forming the second nuclear medicine image data, in a time-of-flight "TOF" kernel corresponding to each of the LORs defined by three or more of the events.

The generation unit may also generate the first nuclear medicine image data using the LOR with the heaviest weight, among the LORs defined by three or more of the events.

The generation unit may also generate the first nuclear medicine image data using the LOR corresponding to the weight, among the LORs defined by three or more of the events.

The generation unit may also generate the first nuclear medicine image data using the weight as a correction factor.

A nuclear medicine image data generation method provided in one aspect of the present invention acquires gamma ray detection data of an object, determines the weight of each of multiple coincidences based on three or more of events detected within a first time window, in the gamma ray detection data, and generates nuclear medicine image data based on the weight.

BRIEF DESCRIPTION OF THE DRAWINGS

FIG. 1 is a diagram showing a schematic of an exemplary detected singles list combined for all detector modules over an exemplary time period.

FIG. 2A is a diagram showing a transaxial cross-sectional schematic of an exemplary positron emission tomography (PET) scanner according to an embodiment.

FIG. 2B shows a transaxial cross-sectional schematic of the exemplary PET scanner including reconstructed image data (PET image data).

FIG. 2C is a diagram showing a transaxial cross-sectional schematic of the exemplary PET scanner including the reconstructed image data, and a first time-of-flight (TOF) kernel and a second TOF kernel.

FIG. 3 is a diagram showing a non-limiting example of a flow chart for a guided pairing method of determining the weighted pairs in a detection window from among a given set of singles within the detection window, according to one exemplary embodiment.

FIG. 4A is a diagram showing a graph of the time difference distribution for a real centered line source, according to one exemplary embodiment.

FIG. 4B is a diagram showing a zoom of the peak for the graph of the time difference distribution shown in FIG. 4A, according to one exemplary embodiment.

FIG. 4C is a diagram showing a zoom of the baseline for the graph of the time difference distribution shown in FIG. 4A, according to one exemplary embodiment.

FIG. 5A is a diagram showing an exemplary graph of the time difference distribution for a real centered line source, according to one exemplary embodiment.

FIG. 5B is a diagram showing a zoom of the peak for the exemplary graph of the time difference distribution of FIG. 5A, according to one exemplary embodiment.

FIG. 5C is a diagram showing a zoom of the baseline for the exemplary graph of the time difference distribution of FIG. 5A, according to one exemplary embodiment.

FIG. 6 is a diagram showing a graph of mean and standard deviation for the exemplary prompt events and the exemplary delay events.

FIG. 7A is a diagram showing a non-limiting example of a PET device that can implement the method according to the embodiment.

FIG. 7B is a diagram showing a non-limiting example of a PET device that can implement the method according to the embodiment.

FIG. 7C is a diagram showing an example of a functional configuration of a processor according to the embodiment.

## DETAILED DESCRIPTION

The following disclosure provides many different embodiments, or examples, for implementing different features of the provided subject matter. Specific examples of components and arrangements are described below to simplify the present disclosure. These are, of course, merely examples and are not intended to be limiting. For example, the formation of a first feature over or on a second feature in the description that follows may include embodiments in which the first and second features are formed in direct contact, and may also include embodiments in which additional features may be formed between the first and second features, such that the first and second features may not be in direct contact. In addition, the present disclosure may repeat reference numerals and/or letters in the various examples. This repetition is for the purpose of simplicity and clarity and does not in itself dictate a relationship between the various embodiments and/or configurations discussed.

The order of discussion of the different steps as described herein has been presented for clarity sake. In general, these steps can be performed in any suitable order. Additionally, although each of the different features, techniques, configurations, etc. herein may be discussed in different places of this disclosure, it is intended that each of the concepts can be executed independently of each other or in combination with each other. Accordingly, the present invention can be embodied and viewed in many different ways.

According to one embodiment discussed herein, a method for guided pairing of coincidences includes applying weights to all possible coincidences in a multi-photon coincidence event. Notably, the weight applied can be generated based on pairs of 2-photon coincidence events or based on reconstructed image data (PET image data) generated from the 2-photon coincidence events.

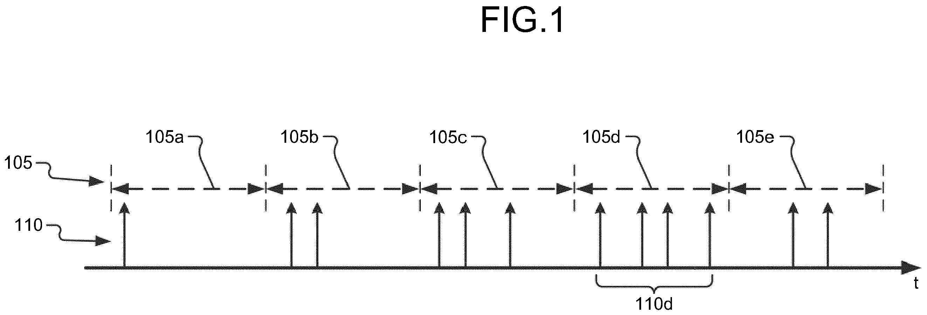

FIG. 1 is a diagram showing a schematic of an exemplary detected singles list combined for all detector modules over an exemplary time period. In an embodiment, a single 110 can be detected at a detector module within a predetermined length of time, or a detection window 105. The detector window is also referred to as a time window. A single is also referred to as a single event or an event. For more than one single 110 detected, a singles list can be compiled showing the detected singles 110 as a function of time. As shown, all of the singles 110 can be separated according to a series of detection windows 105 (in the example of FIG. 1, 105a to 105e). For example, the singles list shown can include a first detection window 105a including one of a plurality of the singles 110, a second detection window 105b including two of the singles 110, a third detection window 105c including three of the singles 110, a fourth detection window 105d including four of the singles 110, and a fifth detection window 105e including two of the singles 110. It may be appreciated that the predetermined length of time of the individual detection windows 105a-105e can vary and be set according to the imaging system (e.g., PET device) used or based on an operator need/desire. In general, the length of time of the individual detection windows 105a-105e will be equal to one another and can be, for example, 12 ns each. Furthermore, it may be appreciated that the predetermined length of time of the individual detection windows 105a-105e generally result in a majority of the detection windows 105a-105e including two of the singles 110, while a minority include one or more than two of the singles 110. However, as previously described, increasing the count rate can cause there to be an increase in the number of the detection windows 105a-105e including more than two of the singles 110 and thereby increasing the rejection rate of some methods attempting to pair the singles. The method described herein attempts to reduce this rejection rate. Consequently, PET image data (reconstructed image data) with good image quality can be generated. The PET image data is an example of nuclear medicine image data. Additionally, the singles list described above is an example of gamma-ray detection data of an object.

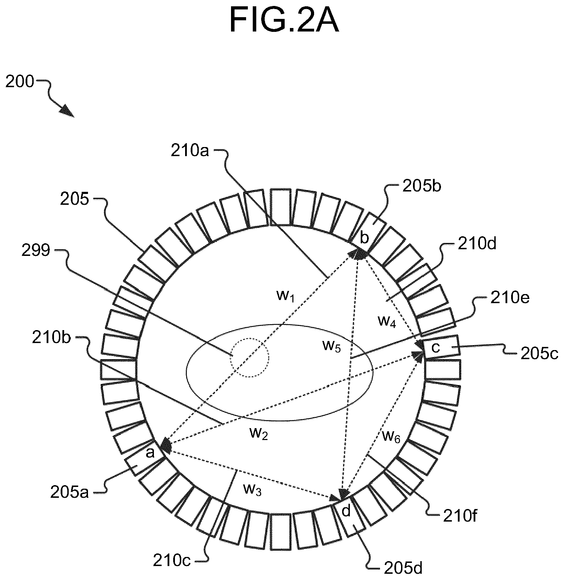

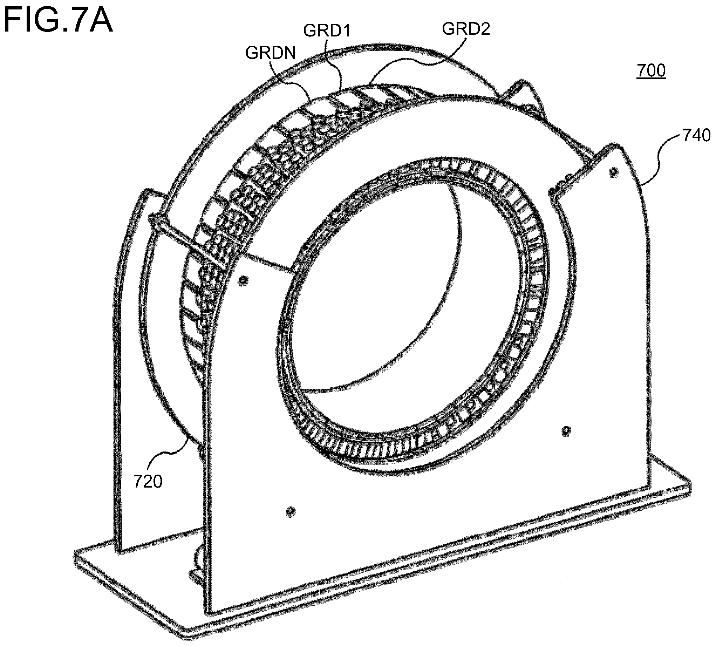

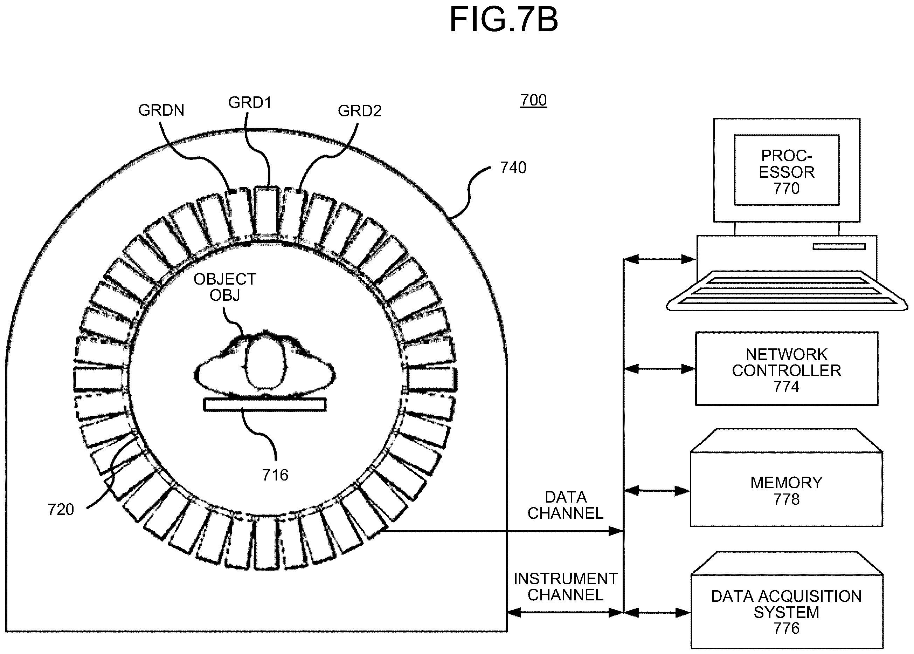

FIG. 2A is a diagram showing a transaxial cross-sectional schematic of an exemplary positron emission tomography (PET) scanner 200 according to an embodiment. For example, a PET device according to the present embodiment includes the PET scanner 200. The PET device is an example of a nuclear medicine diagnosis device. In the exemplary embodiment, the PET scanner 200 includes detector elements 205 arranged in a ring around a central axis that are configured to detect electromagnetic radiation, such as gamma rays. The PET scanner 200 can include additional rings of detector elements 205 disposed along the axis of the rings. Additional features of the PET scanner are shown in FIG. 7A and 7B and will be described below. An object to be scanned such as a phantom or a human can be arranged in the center of the detector elements 205. The object can include a high count-emitting region 299, such as a human heart or lung deemed of high importance during imaging.

When an emitted positron from the phantom or human collides with an electron, an annihilation event occurs, wherein the positron and electron are combined. Most of the time, the annihilation event produces two gamma rays (at 511 keV) traveling at substantially 180 degrees apart. One of these gamma rays is referred to as the single 110. To reconstruct the spatio-temporal distribution of tracers via tomographic reconstruction principles, each detected event is characterized for its energy (i.e., amount of light generated), its location, and its timing. By detecting the two gamma rays (i.e., two of the singles 110), and drawing a line between their locations (i.e., by calculating a line of response (LOR)), one can determine the likely location of the original disintegration.

In one example shown in FIG. 1, during the fourth detection window 105d, four of the singles 110 are detected. That subset will be referred to herein as "the singles subset" 110d. An exemplary corresponding process of spatially detecting the singles subset is shown in FIG. 2A. All possible combinations of pairing the singles subset 110d are shown wherein pairs of two of the singles subset 110d traveling at substantially 180 degrees apart from an annihilation event are grouped. Grouped pairs of singles can be referred to as a 2-photon coincidence 210 or simply as a "coincidence 210". For example, a first detector element 205a can detect a first singles subset (labeled "a") of the singles subset 110d, a second detector element 205b can detect a second singles subset (labeled "b") of the singles subset 110d, a third detector element 205c can detect a third singles subset (labeled "c") of the singles subset 110d, and a fourth detector element 205d can detect a fourth singles subset (labeled "d") of the singles subset 110d. Thus, for pairing the singles subset 110d, a combinatorics formula (combination) of "N Choose 2" or $NC_2$ can be used to determine the maximum possible number of the 2-photon coincidences 210. In this example, N is the number of singles 110 detected in one detection window 105. Additionally, "N Choose 2" or $NC_2$ is the number of methods (combinations) used for selecting two singles 110 from N different singles 110. Concomitantly to the singles subset, for all four of the singles subset 110d, a maximum possible number of the coincidences 210 is six. In the same example as shown, the six possible coincidences 210 are labeled such that: a first coincidence 210a is the coincidence between detector elements "a" and "b", a second coincidence 210b is the coincidence between detector elements "a" and "c", a third coincidence 210c is the coincidence between detector elements "a" and "d", a fourth coincidence 210d is the coincidence between detector elements "b" and "c", a fifth coincidence 210e is the coincidence between detector elements "b" and "d", and a sixth coincidence 210f is the coincidence between detector elements "c" and "d".

In conventional PET scanners, more than two singles 110 detected in the same detection window, such as the fourth detection window 105d, would lead to rejection of the data since a true coincidence (i.e., the coincidence 210 having the true origin of the annihilation along the LOR) cannot be determined. Alternatively, more than two detected singles 110 are all accepted.

To keep all of the singles 110 in an event having detected more than two singles 110, a first detected single is paired with all possible subsequent detected singles that fall within the detection window 105. Then the same pairing process is iterated for the next detected single within the detection window 105 until all detected singles in the detection window 105 are exhausted. This process is iterated for all the remaining detected singles until all detected singles in the detected singles list are exhausted.

To reject all of the singles 110 in the event having detected more than two singles 110, the first detected single is counted along with all subsequent singles falling within the same detection window 105. Upon determining only one other of the single 110 exists in the detection window 105, the two singles 110 are paired and determined to be the one possible coincidence. Upon determining more than one other of the single 110 exists in the detection window 105, all of the singles 110 are skipped without any pairing. The process is then moved to start at the first detected single in a subsequent, new detection window 105. This process is iterated until all detected singles in the detected singles list are exhausted.

However, wholly accepting or rejecting all of the singles 110 without attempting to determine the true coincidences can lead to degradation of image quality and data. Therefore, described herein is a method for determining the true coincidences from a detection event for multiple of the singles 110 while rejecting random coincidences to improve the quality of reconstructed image data (for example, nuclear medicine image data such as PET image data). While previous methods of accepting and rejecting may be based solely on timing information of the single 110, the method described herein uses guided pairing processes based on applied weights. The weight can be based on the probability of the coincidence in question being the true coincidence. All of the coincidences (All LORs) can be assigned a weight and considered before determining to accept or reject said coincidence in question, which can then be used for final image reconstruction.

Referring again to FIG. 2A, the singles subset 110d are detected by the four detector elements 205 to generate the six coincidences 210a-210f. In general, there can be N detected singles, leading to N Choose 2 $(NC_2)$ possible coincidences. In an embodiment, a weight, $w$, can be assigned to each of the six coincidences 210a-210f. Assigning weight w to the coincidence 210 and assigning weight w to the LOR corresponding to the coincidence 210 have the same meaning. As shown, $w_1$ is assigned to the first coincidence 210a, and $w_2$ is assigned to the second coincidence 210b. Additionally, as shown in FIG. 2A, $w_3$, $w_4$, $w_5$, and $w_6$ are respectively assigned to the third coincidence 210c, fourth coincidence 210d, fifth coincidence 210e, and sixth coincidence 210f. To obtain the weights $w_1$ to $w_6$ for the six example coincidences 210a-210f, a number of counts $p_i$ detected along each of the six coincidences 210a-210f can be determined. That is to say, the number of counts $p_i$ detected can be represented by the number of the detection windows 105 including exactly two of the singles 110 detected.

Then, the weight $w_i$ can be determined according to the following equation (1).

$$w_i = \frac{1}{T} p_i \qquad (1)$$

In equation (1), $T$ is a normalization factor to make the sum of all weights $w_i$ to be one, as shown in the following equation (2).

$$\sum_1^{NC_2} w_i = 1 \qquad (2)$$

Notably, as the first coincidence 210a passes through the high count-emitting region 299, the value of $p_1$ will be higher and therefore the weight $w_1$ of the first coincidence 210a will be concomitantly higher. For example, a case when there are one hundred of the detection windows 105 in which exactly two of the singles 110 are detected, and in ninety of the one hundred cases, the two singles 110 are detected at the first detector element 205a and the second detector element 205b will be described. That is, a case when the first coincidence 210a form ninety counts will be described. In this case, there is a majority of counts detected forming the first coincidence 210a and therefore the first coincidence 210a can have a higher weight. It may be appreciated that empirically, such a first coincidence 210a may pass through a region closer to the center of the object and a high-value region (such as lungs, the heart, etc.), the second coincidence 210b may pass through a region farther from the center of the object but potentially through a high-value region (such as the lungs), and the fifth coincidence 210e may pass through a region closer to the periphery of the object and a lower-value region (such as the shoulders). Additionally, it may be appreciated that the third coincidence 210c, fourth coincidence 210d, and sixth coincidence 210f do not pass through the object at all. Thus, empirically, it is assumed that the value of the weights is determined to be : $w_1 > w_2 > w_5 > w_3 > w_4 > w_6$.

Thus, in the example shown in FIG. 2A, in the singles list, the weight $w_i$ of each of the multiple coincidences 210 (first coincidence 210a to sixth coincidence 210f) is determined based on more than two singles 110 detected within the detection windows 105. The detection window 105 in which more than two singles 110 are detected is an example of a first detection window. Additionally, in the example shown in FIG. 2A, the weight $w_i$ is determined based on the LOR defined by the two singles 110 within the detection window 105 including only the two of the singles 110. Specifically, the weight $w_i$ is determined based on the frequency of the LOR defined by the two singles 110 within the detection window 105 including only the two of the singles 110. More specifically, the weight $w_i$ is determined such that the weight $w_i$ increases as the frequency increases.

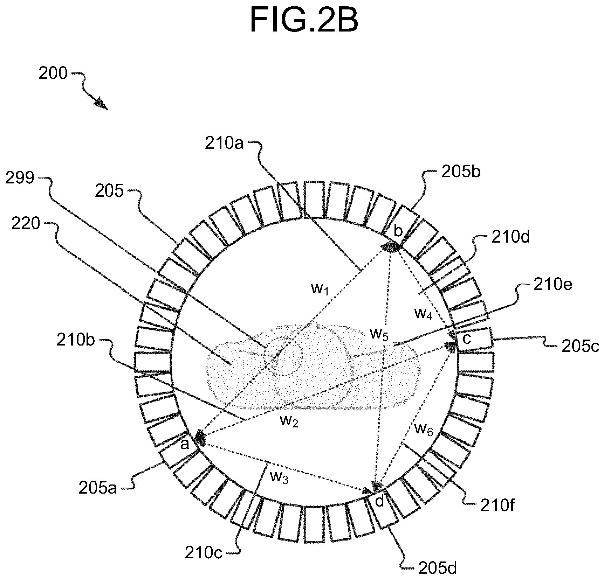

FIG. 2B shows a transaxial cross-sectional schematic of the exemplary PET scanner 200 including reconstructed image data (PET image data) 220. In one embodiment, the weights $w_1$ to $w_6$ can be determined by using the reconstructed image data 220 generated (reconstructed) by performing a PET image reconstruction based on the detection windows 105 including only said two of the singles 110. A random and scatter correction can be applied. However, the reconstructed image data 220 can exclude an attenuation correction. Subsequently, in the example shown in FIG. 2B, the weight $w_i$ can be determined according to the following equation (3).

$$w_i = \frac{1}{T}\ (HX)_j\ \ (3)$$

In equation (3), $H$ is the system matrix, $X$ is the reconstructed image data 220, j is the index of the corresponding coincidence 210 of the whole system (whole PET device), and T is the normalization factor. That is, the weight $w_i$ of the coincidence 210 is determined based on the forward projection of the reconstructed image data 220 to the LOR of the coincidence 210. Notably, the high count-emitting region 299 can be confirmed to be a region of high importance for imaging (e.g., a patient's heart), thus further confirming the value of weights to be: $w_1 > w_2 > w_5 > w_3 > w_4 > w_6$.

In the example shown in FIG. 2B, for example, because the accumulation of the agent can be detected from the reconstructed image data 220, the weight $w_i$ is determined such that the weight $w_i$ of the LOR that passes through the highly accumulated region is large. Thus, in the example shown in FIG. 2B also, in the singles list, the weight $w_i$ of each of the multiple coincidences 210 (first coincidence 210a to sixth coincidence 210f) is determined based on more than two singles 110 detected within the detection windows 105. Specifically, the weight $w_i$ is determined based on the reconstructed image data 220 generated based on the LOR defined by the two singles 110. More specifically, for example, the weight $w_i$ of each of the coincidences 210 is determined based on the intensities of voxels forming the reconstructed image data 220 and through which each of a plurality of the LORs defined by more than two singles 110 detected within the detection window 105 pass. The reconstructed image data 220 is an example of second nuclear medicine image data.

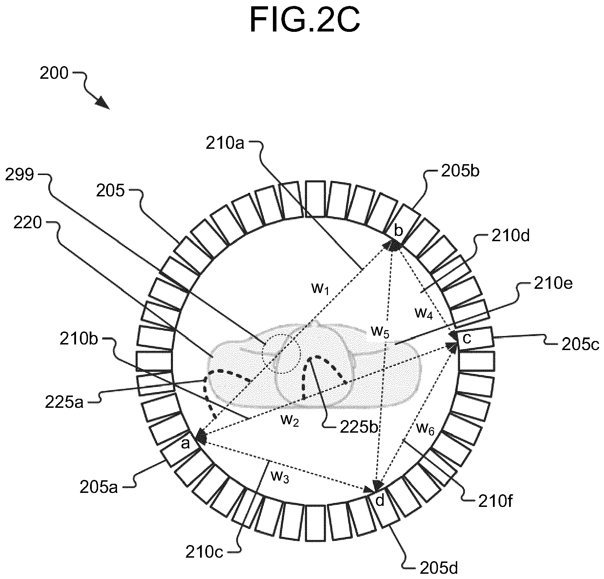

FIG. 2C is a diagram showing a transaxial cross-sectional schematic of the exemplary PET scanner 200 including the reconstructed image data 220 and a first time-of-flight (TOF) kernel 225a and a second TOF kernel 225b. In one embodiment, the reconstructed image data 220 shown in FIG. 2C is generated by the same method as that for generating the reconstructed image data 220 shown in the example of FIG. 2B. A random and scatter correction can be applied. However, the reconstructed image data 220 can exclude an attenuation correction. The weights $w_1$ to $w_6$ can be determined by the forward projection of the reconstructed image data 220 within a range of the TOF kernels 225a-225b of the coincidence 210 in question. For each possible pair in FIG. 2C, there is a timing difference between the two events (the singles 110 detected at the two detectors "a" and "b"). When the weight $w_1$ for the first coincidence 210a is determined, only the image data convoluted within the TOF kernel range will be used for the calculation. That is, in the example shown in FIG. 2C, the weight $w_i$

can be determined according to the following equation (4).

$$w_i = \frac{1}{T}\ (HX^{TOF})_j\ \ (4)$$

In equation (4), $H$ is the system matrix, $X^{TOF}$ is the reconstructed image data 220 convolved with the TOF kernels 225a-225b, j is the index of the corresponding coincidence 210 of the whole system (whole PET device), and T is the normalization factor. Notably, the TOF kernels 225a-225b can further identify the potential origination location of the annihilation event of the coincidence 210. This leads to a significant increase in accuracy as explained as follows.

Thus, in the example shown in FIG. 2C also, in the singles list, the weight $w_i$ of each of the multiple coincidences 210 (first coincidence 210a to sixth coincidence 210f) is determined based on more than two singles 110 detected within the detection windows 105. Specifically, the weight $w_i$ is determined based on the reconstructed image data 220 generated based on the LOR defined by the two singles 110. More specifically, for example, the weight $w_i$ of each of the coincidences 210 is determined based on the intensities of voxels forming the reconstructed image data 220, in the TOF kernel corresponding to each of the LORs.

In an example, the value of weight $w_1$ is greater than weight $w_2$ for FIG. 2B since the first coincidence 210a appears to pass through the high count-emitting region 299. The same appears to be true for FIG. 2C, however, the first TOF kernel 225a depicted in FIG. 2C provides further clarifying information that the potential origin of the annihilation event of the first coincidence 210a is actually located outside the high count-emitting region 299. Furthermore, the first TOF kernel 225a is actually located only partially within the human's body in the reconstructed image data 220 and even partially outside the body altogether. In contrast, the second TOF kernel 225b can be located within the human's body and nearly passing through the center of mass. Without the TOF information, the relative weights would be $w_1 > w_2 > w_5$, and there are more detected counts along the first coincidence 210a as compared to the second coincidence 210b. That is, in the non-TOF scenario, a sum of the image voxel intensities can be calculated which pass through each of the coincidences 210a, 210b, and 210e. Then from this image data, it can be determined that the sum of the voxel intensities for the first coincidence 210a is larger than sum of the second coincidence 210b and the sum of the fifth coincidence 210e since the first coincidence 210a has a very high count activity from the high count-emitting region 299 (e.g., the heart). Instead, upon introduction of the first TOF kernel 225a and the second TOF kernel 225b (and any other necessary TOF kernels 225 for any other of the coincidences 210), the timing difference elucidates that the location of the origin of the first coincidence 210a would have been mostly outside the body. Furthermore, the sum of the voxel intensities only within the first TOF kernel 225a or the second TOF kernel 225b can be summed. Thus, if the voxel intensities within the second TOF kernel 225b are larger than those of the first TOF kernel 225a, then the weight will be larger, and it can be determined that the opposite is true. That is, the weights are determined to be $w_1 < w_5 < w_2$.

Once all weights $w_i$ have been assigned to each of the coincidences 210 (LORs), the weights can be used for guided pairing or reconstructions.

In one embodiment, only one coincidence 210 paired for each of the detection windows 105 can be accepted, wherein the coincidence 210 with the highest weight $w$ is paired, and all other coincidences 210 are rejected. For example, in the example of FIG. 2A, $w_1 > w_2 > w_5 > w_3 > w_4 > w_6$. Thus, only the first coincidence 210a having weight $w_1$ is kept and the other coincidences 210b210f are rejected. Then, based on the weight $w_1$, the reconstructed image data (PET image data) is generated. Specifically, the reconstructed image data is generated based on the first coincidence 210a with weight $w_1$. Thus, in one embodiment, in the coincidences 210 (LORs), the reconstructed image data is generated using the coincidence (LOR) with the heaviest weight. Thus, the reconstructed image data generated in this manner is an example of first nuclear medicine image data.

Additionally, in another embodiment, each coincidence 210 is saved by a possibility of the weight $w_i$ or rejected by a possibility of $1-w_i$. In this example, it is $0 < w_i < 1$. For example, the first coincidence 210a can have a weight $w_1$ of 0.75. To determine whether to accept or reject the first coincidence 210a, a number can be generated having a uniform distribution between 0 and 1. If the generated number is less than or equal to 0.75, the event for the first coincidence 210a is accepted, otherwise the event is rejected. For example, the number larger than 0 and smaller than 1 is generated at random, and the generated number and the weight $w_i$ are compared. If the generated number is less than or equal to the weight $w_i$, the coincidence 210 (LOR) assigned with the weight $w_i$ is received. On the other hand, if the generated number is greater than the weight $w_i$, the coincidence 210 (LOR) assigned with the weight $w_i$ is rejected.

Whether to accept or reject the coincidence 210 (LOR) assigned with the weight $w_i$ may be determined by the probability corresponding to the weight $w_i$. For example, if the weight $w_1$ is 0.3, the first coincidence 210a (LOR corresponding to the first coincidence 210a) assigned with the weight $w_1$ may be accepted at probability of 30 % (($w_1 \times 100$)%) according to the weight, and the first coincidence 210a (LOR corresponding to the first coincidence 210a) assigned with the weight $w_1$ may be rejected at probability of 70 % (100- ($w_1 \times 100$)%) .

Thus, in the other embodiment, the reconstructed image data is generated using the coincidence 210 (LOR) according to the weight $w_i$ in the coincidences 210 (LORs). The reconstructed image data generated in this manner is also an example of the first nuclear medicine image data.

Additionally, in further another embodiment, all of the possible coincidences 210 can be saved along with their corresponding weights $w_i$. During sinogram reconstruction, all of the possible coincidences 210 can be multiplied by the corresponding weights $w_i$ to the sinogram. During list-mode reconstruction, the weight $w_i$ for each of the coincidences 210 can be retained, and the weight $w_i$ can be used as a correction factor during iterative reconstruction.

Thus, in further another embodiment, the reconstructed image data is generated using the weight $w_i$ as a correction factor. The reconstructed image data generated in this manner is also an example of the first nuclear medicine image data.

Thus, according to the various embodiments described above, the reconstructed image data is generated using a suitable coincidence 210 (LOR) corresponding to the weight $w_i$, and the reconstructed image data is generated using the weight $w_i$ as a correction factor. Thus, the reconstructed image data with good image quality can be generated.

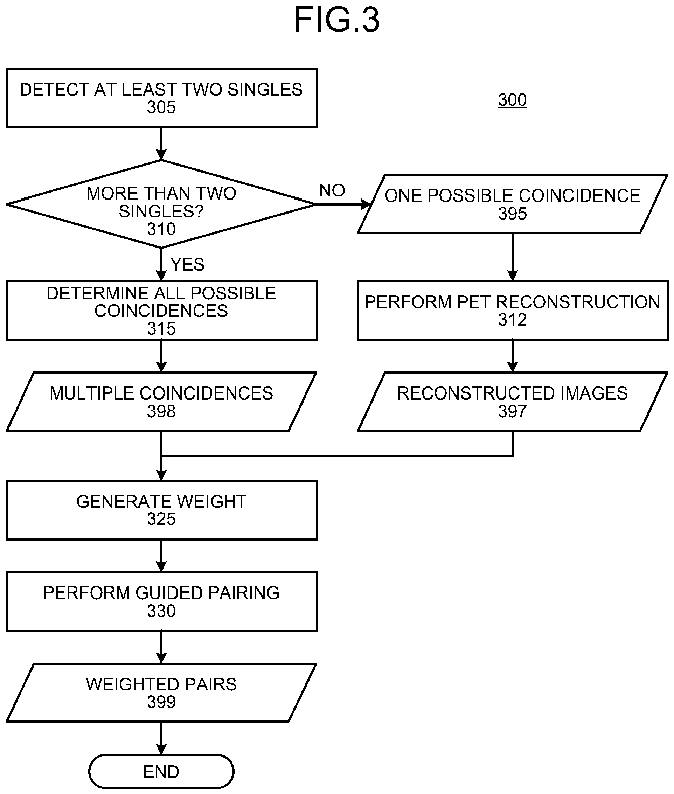

FIG. 3 is a diagram showing a non-limiting example of a flow chart for a guided pairing method 300 of determining the weighted pairs in a detection window from among a given set of singles within the detection window, according to one exemplary embodiment.

In step 305, the PET device (PET system) detects at least two of the singles 110 within the detection window 105. In an embodiment, when one single 110 is detected within the detection window 105, there is no other single 110 with which to pair the detected single 110, and thus the data is not accepted. When at least two singles 110 are detected, the at least two singles 110 can be paired.

In step 310, the PET device determines whether more than two singles 110 are detected within the detection window 105 in step 305. In step 310, if it is determined that more than two singles 110 are not detected within the detection window 105 in step 305 (No in step 310), the two singles 110 are detected in step 305. Thus, the two singles 110 can be paired, and there are no other possible pairings available. Thus, in step 395, one coincidence 395 is derived. In step 312, a PET image reconstruction is performed based on the one possible coincidence 395 to generate reconstructed image data 397.

On the other hand, in step 310, if it is determined that more than two singles 110 are detected within the detection window 105 in step 305 (Yes in step 310), in step 315, all possible pairings of the singles 110 are performed and all possible coincidences 210 are determined from the set of the singles 110 for the detection window 105, and in step 398, all possible multiple coincidences are derived. The maximum number of possible coincidences 210 can be determined via N Choose 2, wherein N is the number of the detected singles 110 in the detection window 105. For example, as seen in FIGs. 2A-2C, six possible coincidences 210 of the four detected singles 110 at detector elements 205a-205d are derived using 4 Choose 2.

In step 325, the weight $w_i$ for each of the coincidences 210 (LORs) is generated. For example, the weight $w_i$ can be based on detected counts or the sum of the voxel intensities along the coincidence 210. Additionally, the weight $w_i$ can be based on the forward projection of the PET image reconstruction of the coincidence 210. For example, the weight $w_i$ can also be based on the forward projection of the reconstructed image within the TOF kernel 225 of the coincidence 210.

In step 330, guided pairing of the singles can be performed based on the weighted coincidences 210 to yield weighted pairs 399. The weighted pairs 399 can then be used in generating an updated reconstructed image. In one embodiment, all of the possible coincidences 210 can be saved along with their corresponding weights $w_i$. During sinogram reconstruction, all of the possible coincidences 210 can be multiplied by the corresponding weights $w_i$ to the sinogram. During list-mode reconstruction, the weight $w_i$ for each of the coincidences 210 can be retained and used as a correction factor during iterative reconstruction.

To obtain quantitative data in PET, the sum of the true and scattered coincidences can be determined by estimating and subtracting the random coincidences from the measured data in each LOR. The number of random coincidences detected as delayed coincidences will equal, on average, the number of random coincidences in the prompt coincidence sinogram. With a correction for random coincidences, the delayed coincidences are subtracted from the prompt coincidences sinogram as they occur. More precisely, using mean values, $P = T + S + R$ and $D = R$, so the correction for random coincidences is $T + S = P - D$, where P, T, S, R, and D are the numbers (or rates) of prompt, true, scattered, random, and delayed coincidences. This provides an accurate correction for random coincidences but also increases statistical noise in the net (prompt) - (delay) coincidence sinogram.

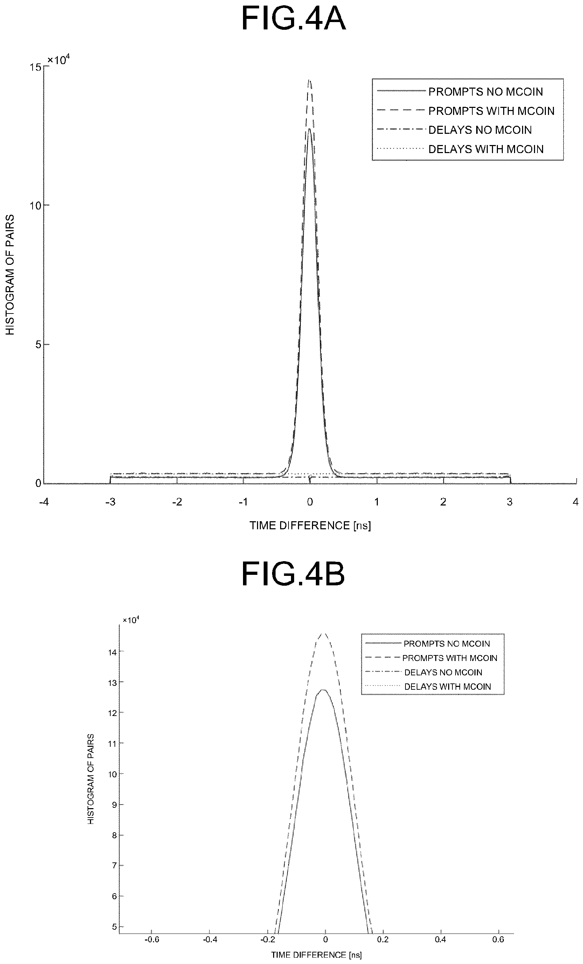

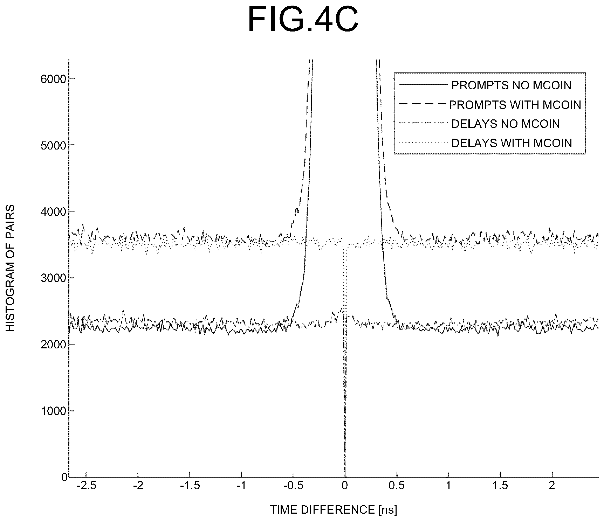

In order to test the effectiveness of the method described, and validate that prompts and delays have the same distributions for random events (also referred to as "randoms"), a real centered line source was used to test the time difference distribution. Prompts can be understood to mean prompt coincidence events, or those detected within the detection window 105. Delays can be understood to mean delayed coincidence events, wherein the detection window 105 is lengthened greatly compared to the detection window 105 of the detected prompt coincidence events. Notably, the possibility rate of a random event being detected within the detection window ($|t1-t2|<tc$, t1 and t1 are the times for the two singles, and tc is the detection window size) is the same as for the random event within a delayed detection window ($|t1-(t2+td)|<tc$), wherein td is a user defined parameter that describes a fixed time difference between two singles. For a real centered line source, a graph of the time difference distribution is shown in FIG. 4A, according to one exemplary embodiment. FIG. 4B is a diagram showing a zoom of the peak for the graph of the time difference distribution shown in FIG. 4A, according to one exemplary embodiment. FIG. 4C is a diagram showing a zoom of the baseline for the graph of the time difference distribution shown in FIG. 4A, according to one exemplary embodiment. Note that FIG. 4A does not include guided pairing results and is presented to demonstrate baseline data for accepting all the coincidences (see "with mcoin") or rejecting all the coincidences (see "no mcoin").

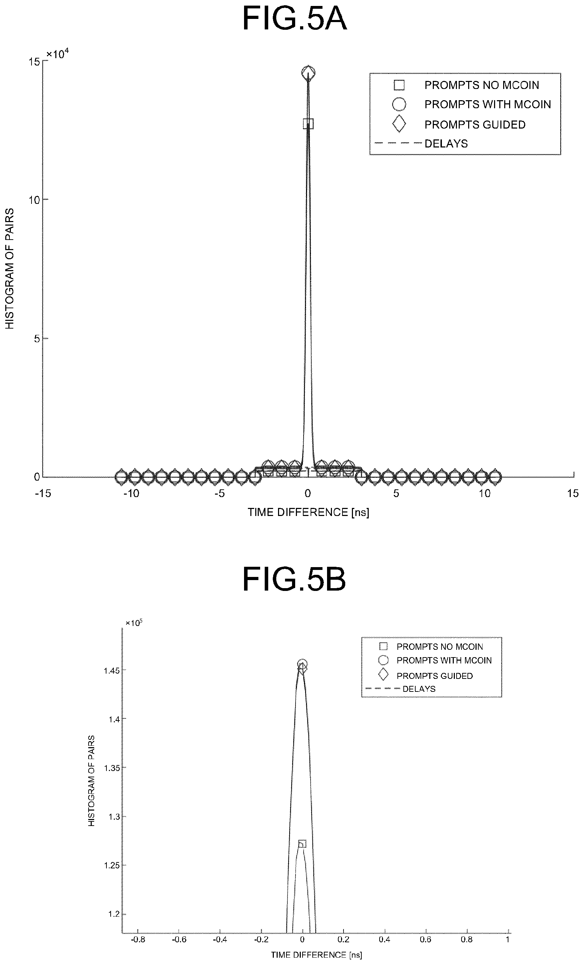

By using the disclosed guided pairing method, more true events are generated and randoms are reduced. Data demonstrating the effectiveness of the disclosed method is shown in FIGs. 5A-5C, including the baseline data of FIGs. 4A-4C.

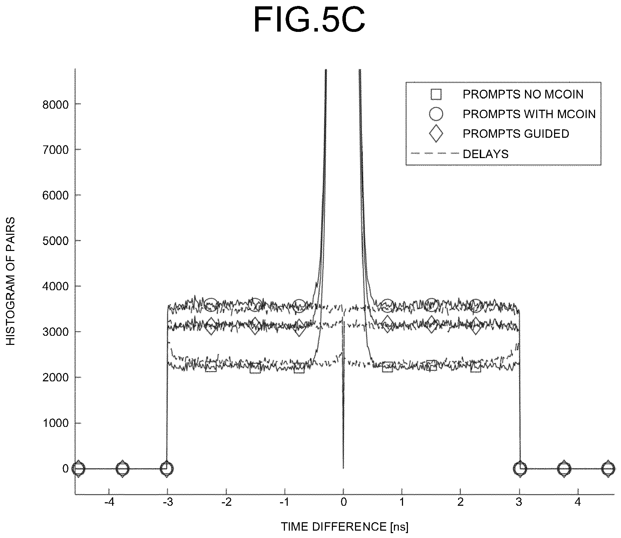

FIG. 5A is a diagram showing an exemplary graph of the time difference distribution for a real centered line source, according to one exemplary embodiment. FIG. 5B is a diagram showing a zoom of the peak for the exemplary graph of the time difference distribution of FIG. 5A, according to one exemplary embodiment. FIG. 5C is a diagram showing a zoom of the baseline for the exemplary graph of the time difference distribution of FIG. 5A, according to one exemplary embodiment. In the above method, guided paring results in the same amount of "prompts" (i.e. true pairs) compared with keeping all the coincidences, thus indicating that the guided pairing can find the trues in the coincidences. Additionally, the amount of prompts via rejecting all coincidences is much lower than both keeping all coincidences or the described guided pairing method. On the other hand, the guided pairing method has less randoms compared with keeping all coincidences.

Notably, the guided pairing results in a similar peak height as keeping all the coincidences in FIG. 5B, while also resulting in a lower baseline as compared to keeping all the coincidences in FIG. 5C. Therefore, this greater difference between peak and baseline demonstrates a higher signal-to-noise ratio by using the guided pairing method as compared to systems using the accept all or reject all coincidences methods. Additionally, the improvement in data quality as described above does not require a significant increase in computation power. However, it should be highlighted that a computational offset can occur wherein rejecting some of the coincidences results in fewer pairs for reconstruction, thus leading to faster reconstruction time. Therefore, as demonstrated, the disclosed method and system herein provides advantages over current imaging systems, including improved accuracy of identifying the true coincidences, improved SNR and data quality, and faster processing and reconstruction time.

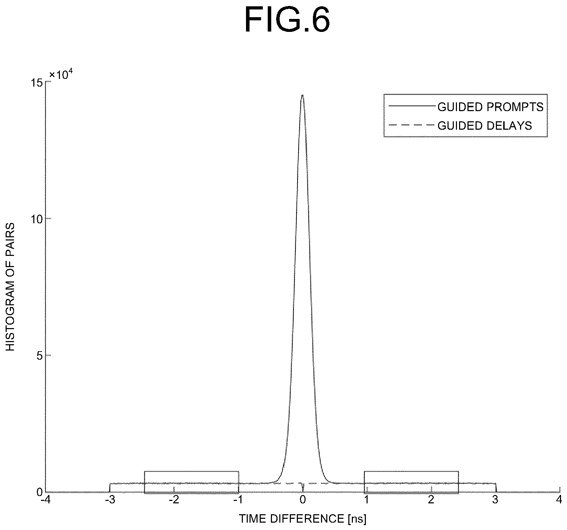

FIG. 6 is a diagram showing a graph of mean and standard deviation for exemplary prompt events and exemplary delay events. To test if there is a bias introduced in the guided pairs data, the mean and standard deviation were calculated for the prompt and delay events within the two windows as shown in FIG. 6 with dashed boxes. The two windows include 200 data points, and result in a mean $\pm$ standard deviation of: $N_{pp}$ = 3167 $\pm$ 59 (prompt) and $N_{de}$ = 3124 $\pm$ 57 (delay). From this, the following equation (5) and equation (6) can be derived.

$$\Delta N = N_{pp} - N_{de} = 43 \qquad (5)$$

$$\sigma(\Delta N) = \sqrt{59^2 + 57^2} = 82 \qquad (6)$$

where $N_{pp}$ is the mean number of prompt points, $N_{de}$ is the mean number of delay points, $\Delta N$ is a difference between the mean number of prompt points and the mean number of delay points, and $\sigma(\Delta N)$ is the standard deviation of $\Delta N$. Notably, since $\Delta N < \sigma(\Delta N)$, the two tail regions in the black boxes do not have a significant difference, and therefore, no bias is introduced.

FIGs. 7A and 7B are diagrams each showing a non-limiting example of a PET device 700 that can implement the method 300 according to the embodiment. The PET device 700 includes a detector ring including a number of gamma-ray detectors (GRDs) (e.g., GRD1, GRD2, through GRDN) that are each configured as rectangular detector modules. According to one implementation, the detector ring includes 40 GRDs. In another implementation, there are 48 GRDs, and the higher number of GRDs is used to create a larger bore size for the PET device 700. The PET device 700 is an example of the nuclear medicine diagnosis device.

Each GRD can include a two-dimensional array of individual detector crystals, which absorb gamma radiation and emit scintillation photons. The scintillation photons can be detected by a two-dimensional array of photomultiplier tubes (PMTs) that are also arranged in the GRD. A light guide can be disposed between the array of detector crystals and the PMTs.

Alternatively, the scintillation photons can be detected by an array of silicon photomultipliers (SiPMs), and each individual detector crystals can have a respective SiPM.

Each photodetector (e.g., PMT or SiPM) can produce an analog signal that indicates when scintillation events have occurred, and an energy of the gamma ray producing the detection event. Moreover, the photons emitted from one detector crystal can be detected by more than one photodetector, and, based on the analog signal produced at each photodetector, the detector crystal corresponding to the detection event can be determined using Anger logic and crystal decoding, for example.

FIG. 7B shows a schematic view of a PET device (PET scanner system) having gamma-ray photon counting detectors (GRDs) arranged to detect gamma-rays emitted from an object OBJ. The GRDs can measure the timing, position, and energy corresponding to each gamma-ray detection. In one implementation, the gamma-ray detectors are arranged in a ring, as shown in Figures 7A and 7B. The detector crystals can be scintillator crystals, which have individual scintillator elements arranged in a two-dimensional array and the scintillator elements can be any known scintillating material. The PMTs can be arranged such that light from each scintillator element is detected by multiple PMTs to enable Anger arithmetic and crystal decoding of scintillation event.

FIG. 7B shows an example of the arrangement of the PET device 700, in which the object OBJ to be imaged rests on a table 716 and the GRD modules GRD1 through GRDN are arranged circumferentially around the object OBJ and the table 716. The GRDs can be fixedly connected to a circular component 720 that is fixedly connected to the gantry 740. The gantry 740 houses many parts of the PET device 700. The gantry 740 of the PET device 700 also includes an open aperture through which the object OBJ and the table 716 can pass, and gamma-rays emitted in opposite directions from the object OBJ due to an annihilation event can be detected by the GRDs, and timing and energy information can be used to determine coincidences for gamma-ray pairs.

In FIG. 7B, circuitry and hardware is also shown for acquiring, storing, processing, and distributing gamma-ray detection data. As the circuitry and hardware, the PET device 700 includes a processor 770, a network controller 774, a memory 778, and a data acquisition system (DAS) 776. The PET device 700 also includes a data channel that routes detection measurement results from the GRDs to the DAS 776, the processor 770, the memory 778, and the network controller 774. The DAS 776 can control the acquisition, digitization, and routing of the detection data from the detectors. In one embodiment, the DAS 776 controls the movement of the table 716. The processor 770 performs functions including reconstruction of reconstructed image data from the detection data (e.g., the singles list described above), prereconstruction processing of the detection data, and postreconstruction processing of the image data.

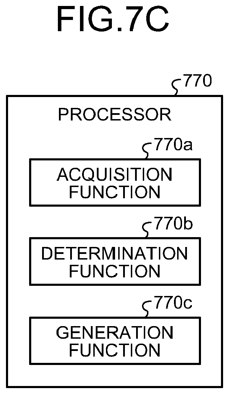

The processor 770 can be configured to perform various steps of the method 300 described herein, variations thereof, and various processes described above. FIG. 7C is a diagram showing an example of a functional configuration of the processor 770 according to the embodiment. As shown in FIG. 7C, the processor 770 includes an acquisition function 770a, a determination function 770b, and a generation function 770c. The processing functions of the acquisition function 770a, the determination function 770b, and the generation function 770c that are components of the processor 770 are recorded in memory 778 of the PET device 700 in the form of computer-executable programs. The processor 770 is a processor that implements a processing function corresponding to each computer program, by reading out the computer program from the memory 778 and executing the read computer program. In other words, the processor 770 that has read out the computer programs has the processing functions shown in the processor 770 of FIG. 7C.

The acquisition function 770a generates the singles list described above from the detection measurement results from the GRDs collected by the DAS 776. The acquisition function 770a acquires the singles list in this manner. The acquisition function 770a is an example of an acquisition unit.

The determination function 770b determines the weight $w_i$ using the singles list acquired by the acquisition function 770a, and performs various processes of assigning the weight $w_i$ to the coincidence 210 (LOR) described above. The determination function 770b is an example of a determination unit.

The generation function 770c performs various processes of generating the reconstructed image data described above, using the weight $w_i$. The generation function 770c is an example of a generation unit.

The term "processor" used in the explanation is, for example, a Central Processing Unit (CPU), a Graphics Processing Unit (GPU), an Application Specific Integrated Circuit (ASIC), or a programmable logic device (for example, Simple Programmable Logic Device (SPLD) and Complex Programmable Logic Device (CPLD)), or a circuitry such as a Field Programmable Gate Array (FPGA). The processor 770 implements the function by reading out the computer program stored in the memory 778, and executing the read computer program. In addition, instead of storing computer programs in the memory 778, the computer programs may be directly incorporated into the circuitry of the processor 770. In this case, the processor 770 implements the function by reading out and executing the computer program incorporated into the circuitry.

The memory 778 can be a hard disk drive, CD-ROM drive, DVD drive, FLASH drive, RAM, ROM or any other electronic storage known in the art.

The network controller 774, such as an Intel Ethernet (registered trademark) PRO network interface card from Intel Corporation of America, can interface between the various parts of the PET imaging device. Additionally, the network controller 774 can also interface with an external network. As can be appreciated, the external network can be a public network, such as the Internet, or a private network such as an LAN or WAN network, or any combination thereof and can also include PSTN or ISDN sub-networks. The external network can also be wired, such as an Ethernet network, or can be wireless such as a cellular network including EDGE, 3G, 4G, and 5G wireless cellular systems. The wireless network can also be WiFi, Bluetooth (registered trademark), or any other wireless form of communication that is known.

In the preceding description, specific details have been set forth, such as a particular geometry of a processing system and descriptions of various components and processes used therein. It should be understood, however, that techniques herein may be practiced in other embodiments that depart from these specific details, and that such details are for purposes of explanation and not limitation. Embodiments disclosed herein have been described with reference to the accompanying drawings. Similarly, for purposes of explanation, specific numbers, materials, and configurations have been set forth in order to provide a thorough understanding. Nevertheless, embodiments may be practiced without such specific details. Components having substantially the same functional constructions are denoted by like reference characters, and thus any redundant descriptions may be omitted.

Various techniques have been described as multiple discrete operations to assist in understanding the various embodiments. The order of description should not be construed as to imply that these operations are necessarily order dependent. Indeed, these operations need not be performed in the order of presentation. Operations described may be performed in a different order than the described embodiment. Various additional operations may be performed and/or described operations may be omitted in additional embodiments.

According to at least one of the embodiments described above, a nuclear medicine image data with good image quality can be generated.

While several embodiments have been described, these embodiments are presented as examples and are not intended to limit the scope of the invention. These embodiments can be implemented in various other forms, and it is possible to perform various omissions, replacements, and changes without departing from the scope of the invention as defined by the appended claims.

**Claims**

1. A nuclear medicine diagnosis device, comprising:

   an acquisition unit (770a) configured to acquire gamma ray detection data of an object;
   a determination unit (770b) configured to determine weight of each of multiple coincidences based on three or more of events detected within a first time window, in the gamma ray detection data; and
   a generation unit (770c) configured to generate first nuclear medicine image data based on the weight.

2. The nuclear medicine diagnosis device according to claim 1, wherein the determination unit (770b) is configured to determine the weight based on a line of response "LOR" defined by the two events within a second time window including the two events.

3. The nuclear medicine diagnosis device according to claim 2, wherein the determination unit (770b) is configured to determine the weight based on frequency of the LOR defined by the two events.

4. The nuclear medicine diagnosis device according to claim 3, wherein the determination unit (770b) is configured to determine the weight such that the weight increases as the frequency increases.

5. The nuclear medicine diagnosis device according to claim 2, wherein the determination unit (770b) is configured to determine the weight based on second nuclear medicine image data generated based on the LOR defined by the two events.

6. The nuclear medicine diagnosis device according to claim 5, wherein the determination unit (770b) is configured to determine the weight of each of the coincidences based on intensity of a voxel forming the second nuclear medicine image data and through which each of a plurality of the LORs defined by three or more of the events passes.

7. The nuclear medicine diagnosis device according to claim 5, wherein the determination unit (770b) is configured to determine the weight of each of the coincidences based on intensity of a voxel forming the second nuclear medicine image data, in a time-of-flight "TOF" kernel corresponding to each of a plurality of the LORs defined by three or more of the events.

8. The nuclear medicine diagnosis device according to claim 3, wherein the generation unit (770c) is configured to generate the first nuclear medicine image data using the LOR with heaviest weight, among a plurality of the LORs defined by three or more of the events.

9. The nuclear medicine diagnosis device according to any preceding claim, wherein the generation unit (770c) is configured to generate the first nuclear medicine image data using the LOR corresponding to the weight, among a plurality of the LORs defined by three or more of the events.

10. The nuclear medicine diagnosis device according to any of claims 1 to 8, wherein the generation unit (770c) is configured to generate the first nuclear medicine image data using the weight as a correction factor.

11. A nuclear medicine image data generation method, comprising:

acquiring gamma ray detection data of an object (770a);

determining weight of each of multiple coincidences based on three or more of events detected within a first time window, in the gamma ray detection data (770b); and
generating nuclear medicine image data based on the weight (770c).

FIG.1

# FIG.2A

# FIG.2B

# FIG.2C

# FIG.3

```
DETECT AT LEAST TWO SINGLES
305
```

300

```
MORE THAN TWO
SINGLES?
310
```

NO → 

```
ONE POSSIBLE COINCIDENCE
395
```

YES

```
DETERMINE ALL POSSIBLE
COINCIDENCES
315
```

```
PERFORM PET RECONSTRUCTION
312
```

```
MULTIPLE COINCIDENCES
398
```

```
RECONSTRUCTED IMAGES
397
```

```
GENERATE WEIGHT
325
```

```
PERFORM GUIDED PAIRING
330
```

```
WEIGHTED PAIRS
399
```

```
END
```

# FIG.4A

# FIG.4B

EP 4 075 168 A1

# FIG.4C

# FIG.5A

# FIG.5B

# FIG.5C

# FIG.6

EP 4 075 168 A1

# FIG.7A

GRDN  GRD1  GRD2

700

740

720

# FIG.7B

GRDN GRD1 GRD2 <u>700</u>

740

OBJECT
OBJ

716

720

PROC-
ESSOR
770

NETWORK
CONTROLLER
774

MEMORY
778

DATA
CHANNEL

INSTRUMENT
CHANNEL

DATA ACQUISITION
SYSTEM
776

EP 4 075 168 A1

# FIG.7C

770

PROCESSOR

770a

ACQUISITION
FUNCTION

770b

DETERMINATION
FUNCTION

770c

GENERATION
FUNCTION

Europäisches
Patentamt

European
Patent Office

Office européen
des brevets

**EUROPEAN SEARCH REPORT**

Application Number

EP 22 16 8511

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2013/188011 A1 (MASSACHUSETTS INST TECHNOLOGY [US]; LAGE EDUARDO M [US] ET AL.) 19 December 2013 (2013-12-19) * paragraphs [0055], [0081], [0083], [0086], [0089]; figures 2, 5 * | 1,2,9-11 | INV. G01T1/17 G01T1/29 |
| X | LAGE EDUARDO ET AL: "Recovery and normalization of triple coincidences in PET", MEDICAL PHYSICS, AIP, MELVILLE, NY, US, vol. 42, no. 3, 26 February 2015 (2015-02-26), pages 1398-1410, XP012195023, ISSN: 0094-2405, DOI: 10.1118/1.4908226 [retrieved on 1901-01-01] * Methods, 2.B., 3.C.; pages 1,4,9,10 * | 1-8,11 | |

TECHNICAL FIELDS SEARCHED (IPC)

G01T

The present search report has been drawn up for all claims

| Place of search | Date of completion of the search | Examiner |
|---|---|---|
| The Hague | 29 August 2022 | Johnstone, John |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 8511

This annex lists the patent family members relating to the patent documents cited in the above-mentioned European search report.
The members are as contained in the European Patent Office EDP file on
The European Patent Office is in no way liable for these particulars which are merely given for the purpose of information.

29-08-2022

| Patent document cited in search report | Publication date | Patent family member(s) | Publication date |
|---|---|---|---|
| WO 2013188011 A1 | 19-12-2013 | US 2015185339 A1 | 02-07-2015 |
| | | WO 2013188011 A1 | 19-12-2013 |

EPO FORM P0459

For more details about this annex : see Official Journal of the European Patent Office, No. 12/82